Europäisches Patentamt

European Patent Office

Office européen des brevets

(11) Veröffentlichungsnummer: **0 270 905**
**A2**

(19)

(12) # EUROPÄISCHE PATENTANMELDUNG

(21) Anmeldenummer: 87117282.1

(22) Anmeldetag: 24.11.87

(51) Int. Cl.⁴: **C12N 5/02** , C12M 3/00 ,
C12P 21/00

(30) Priorität: 06.12.86 DE 3641824

(43) Veröffentlichungstag der Anmeldung:
15.06.88 Patentblatt 88/24

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Bödeker, Berthold, Dr.
Pahlkestrasse 17
D-5600 Wuppertal 1(DE)
Erfinder: Hewlett, Guy, Dr.
Krutscheider Weg 96
D-5600 Wuppertal 11(DE)
Erfinder: Nerger, Dittmar, Dr.
Bacherhofstrasse 74
D-4150 Krefeld(DE)
Erfinder: Schlumberger, Horst Dieter, Prof. Dr.
Schwartnerstrasse 5
D-5600 Wuppertal 2(DE)

(54) **Verfahren und Vorrichtung zur Kultivierung immobilisierter oder adhärierter Säugerzellen sowie dessen Verwendung bei der Herstellung von Proteinen.**

(57) Die vorliegende Erfindung betrifft ein Verfahren und Vorrichtung zur Kultivierung immobilisierter oder adhärierter Säugerzellen, wobei die Säugerzellen in einer Wachstumskammer immobilisiert vorliegen und über ein geschlossenes Kreislaufsystem ständig mit Medium umspült werden. Separat von der Wachstumskammer ist in dem Kreislauf ein Modul mit einer semipermeablen Membran angeordnet über das die Zuführung der Nährstoffe und/oder die Abführung der Stoffwechselprodukte erfolgt. Die erfindungsgemäße Vorrichtung ist sehr gut geeignet zur kontinuierlichen Kultivierung von immobilisierten oder adhärierten Säugerzellen unter kontrollierten Bedingungen über lange Zeiträume.

EP 0 270 905 A2

0 270 905

BAYER AKTIENGESELLSCHAFT     5090 Leverkusen, Bayerwerk
Konzernverwaltung RP
Patentabteilung             Bu/ABc

Verfahren und Vorrichtung zur Kultivierung immobilisierter oder adhärierter Säugerzellen sowie dessen Verwendung bei der Herstellung von Proteinen

Die vorliegende Erfindung betrifft ein Verfahren und Vorrichtung zur Kultivierung immobilisierter oder adhärierter Säugerzellen, wobei die Säugerzellen in einer Wachstumskammer immobilisiert oder adhäriert vorliegen und über ein geschlossenes Kreislaufsystem ständig mit Medium umspült werden.

Separat von der Wachstumskammer ist in dem Kreislauf ein Modul mit einer semipermeablen Membran angeordnet über das die Zuführung der Nährstoffe und/oder die Abführung der Stoffwechselprodukte erfolgt. Die erfindungsgemäße Vorrichtung ist sehr gut geeignet zur kontinuierlichen Kultivierung von immobilisierten oder adhärierten Säugerzellen unter kontrollierten Bedingungen über lange Zeiträume.

Le A 24 835 - Ausland

Die Vorrichtung kann bei der Herstellung von natürlichen und rekombinanten Proteinen, Antikörpern und Enzymen verwendet werden.

Bei der Kultivierung tierischer Zellen in Bioreaktoren ergeben sich mehrere Probleme:

(a) die Kultur muß mit den Gasen ($O_2$ und $CO_2$) und Nährstoffen wie Glukose, Aminosäuren, Vitaminen, etc. versorgt sein,

(b) Stoffwechselendprodukte wie Laktat, Ammoniak oder überschüssiges gelöstes $CO_2$ müssen entsorgt werden.

Dies limitiert in der Regel jede Kultur tierischer Zellen, sodaß sowohl die erreichbare Zellzahl als auch die Produktkonzentration begrenzt sind.

Eine Kontrolle der Kulturparameter pH und $pO_2$ sowie der Einsatz kompakter Apparate in solchen technischen Kultursystemen führt zu besserem Wachstum und Produktbildung sowie zu reproduzierbareren Kultivierungen, die auch in größeren Maßstab übertragbar sind.

Eine bereits realisierte Möglichkeit, um die Zellkonzentration zu erhöhen, ist die Perfusion, d.h. ein Mediumaustausch mit Zellrückhaltung. Dies wird durch Spinfilter (A.L. van Wezel (1984), Developments biol. Standard. 55, Seite 3-9), hydrophile Membranen oder externe Kreislaufsysteme mit Filtereinheiten (W.R. Tolbert, Y. Feder (1983), Annual Reports on Fermentation Processes, Vol. 6, Seite 35-74) realisiert.

Le A 24 835

Aufgrund der teilweise geringen Produktbildungsraten in tierischen Zellkulturen ergeben sich jedoch selbst in kontinuierlichen Perfusionskulturen, daß aufgrund der Substrat- und Endproduktlimitierungen hohe Mediumumsätze notwendig sind. Die Produkte müssen daher in weiteren Aufarbeitungsschritten konzentriert und später gereinigt werden.

Im Falle einer Kultivierung in serumfreiem Medium bedingen die hohen Durchsatzraten an komplettem Medium bei der Perfusion, daß alle Schutzproteine wie Albumin, Transferrin und Insulin ständig mit dem zulaufenden Medium erneuert werden müssen. Da diese Substanzen sehr teuer sind, führt dies zu unvertretbar hohen Kosten bei der Kultivierung.

Andererseits ist bekannt, daß tierische Zellen in Hohlfasermodulen, ursprünglich für die Nierendialyse eingesetzt, zu hoher Zellzahl anwachsen und im Falle monoklonaler Antikörper auch zu hohen Produktkonzentrationen führen (O.T. Schönherr, P.Y. van Hees, A.M.Y.M. van Os H.W.M. Roelofs, P.T.Y.A. van Gelder, Proceedings of the 7th ESACT-Meeting, Baden bei Wien, 1985 Develop. biol. Standard, im Druck). Dabei werden die Zellen, die sich im extrakapillaren Raum des Moduls befinden, über eine Gleichgewichtsdialyse durch die Hohlfasermembranen mit frischen Substraten versorgt und von dialysierbaren Stoffwechselendprodukten entsorgt. Der Einsatz solcher Module hat aber beträchtliche Nachteile, da

Le A 24 835

- 4 -

(a) der eigentliche Zellkulturraum weder gut durchmischt noch pH- oder $pO_2$-kontrolliert ist,

(b) die Größe der Module begrenzt ist, da Versorgung und Entsorgung der Zellen diffusionslimitiert sind,

(c) die Zellen sauerstofflimitiert sind, da der notwendige Sauerstoff nur über die Hohlfasermembran eingebracht werden kann,

(d) das Produkt aus dem extrakapillaren Raum schwierig zu ernten ist.

Es wurde nun nach einem Kultivierungsverfahren gesucht bei dem sowohl hohe Zelldichten und Produktkonzentrationen als auch eine Verminderung des Durchsatzes an komplettem serumfreiem Medium erreicht wird.

Erfindungsgemäß wird dies erreicht durch Integration eines Dialyse- oder Ultrafiltrationsmoduls in den Kreislauf eines Kultursystems wie dem Opticell Kultursystem. Dieses Kultursystem enthält eine Wachstumskammer für immobilisierte oder adhärierte Zellen. Die Zellen werden unter kontrollierten Bedingungen (Sauerstoff und pH) im Kreislauf ständig mit Medium versorgt (L. Stotz, B.K. Lydersen, G.G. Pugh, M.S. Paris, L.A. Noll, Bio/Technology Jan. (1985), 63.). Die Kontrolle der Kulturbedingungen wird über integrierte Kontroll- und Regelsysteme für $pO_2$ und pH gewährleistet.

Durch die Integration eines Dialyse- oder Ultrafiltrationsmoduls mit einer semipermeablen Membran in den Kreislauf des Kultursystems ergibt sich die Möglichkeit

Le A 24 835

0 270 905

- 5 -

der kontinuierlichen Zuführung von Nährstoffen zum Kulturmedium und auch der kontinuierlichen Abführung von Stoffwechselendprodukten.

Durch die Integration eines Dialyse- oder Ultrafiltrationsmoduls in das Kultursystem auch "interner Kreislauf" genannt ergeben sich die folgenden Vorteile:

(a) Es ist möglich sehr hohe Zellkonzentrationen von $2-5 \times 10^7$ Zellen/ml Medium im internen Kreislauf zu erreichen und über lange Zeit zu halten.

(b) Durch die Akkumulation des Produkts im internen Kreislauf kommt man zu Produktkonzentrationen, die mehr als 50fach über denen undialysierter Kulturen liegen; dies vereinfacht die Endreinigung des Produkts beträchtlich und reduziert außerdem die Volumina an aufzureinigendem Kulturüberstand.

(c) Die hohe Produktkonzentration im internen Kreislauf erlaubt den Einsatz proteinfreier serumfreier Medien, da der protektive Effekt der Schutzproteine nun vom Produkt selbst übernommen wird; dies erhöht die spezifische Produktkonzentration im Kulturmedium noch zusätzlich;

(d) Bei serumfreier Kultivierung braucht das Austauschmedium nur die niedermolekularen Bestandteile zu enthalten, was die Mediumkosten enorm reduziert, da im internen Kreislauf nur noch eine relativ geringe Menge an vollständig supplementiertem Medium, das z.B. Albumin und Transferin enthält, benötigt wird.

<u>Le A 24 835</u>

- 6 -

(e) Durch die Verkleinerung des eigentlichen Produktbildungsvolumens im Falle der Dialyse bzw. durch das kontinuierliche Verfahren bei der Ultrafiltration werden die Produktbildungsraten bezogen auf das Volumen im internen Kreislauf drastisch erhöht. Dadurch reduzieren sich die notwendigen Reaktionsvolumen, sodaß man mit kleineren Bioreaktoren auskommt;

(f) Aufgrund der ständigen Ent- und Versorgung der Zellen sowie der kontrollierten Kulturbedingungen (pH, $pO_2$) bleibt die Kultur über langen Zeitraum in gutem Zustand, was die Voraussetzung für hohe Produktbildungsraten ist;

(g) Über die Wahl der Molekulargewichtsausschlußgrenze des Hohlfasermoduls kann die Qualität des zurückgeführten Mediumanteils vorgegeben werden. Die Ausschlußgrenze kann von <1.000 bis >150.000 Dalton variabel sein. Sie muß so gewählt werden, daß die Membran für Substrate und inhibitorische Stoffwechselendprodukte permeabel, für das Produkt jedoch impermeabel ist. Bevorzugt eingesetzt werden Membranen mit Ausschlußgrenzen von <100.000 wie z.B. 5.000, 10.000, 20.000 oder 50.000;

(h) Bei der Wahl geeigneter Hohlfasermodule und serumfreier Medien können nicht nur Suspensionszellen, die in der Wachstumskammer immobilisiert werden, sondern auch adhärente Zellen eingesetzt werden, ohne daß die Hohlfasermembran durch sich festsetzende, von der Wachstumskammer abgelöste Zellen verstopft wird. Dies wird durch eine hohe Fließgeschwindigkeit im internen Kreislauf begünstigt.

**Le A 24 835**

Zur Durchführung des erfindungsgemäßen Verfahrens bieten sich zwei Alternativen an, die nachstehend am Beispiel des Opticell Kultursystems beschrieben werden:

(a) <u>Gleichgewichtsdialyse</u>

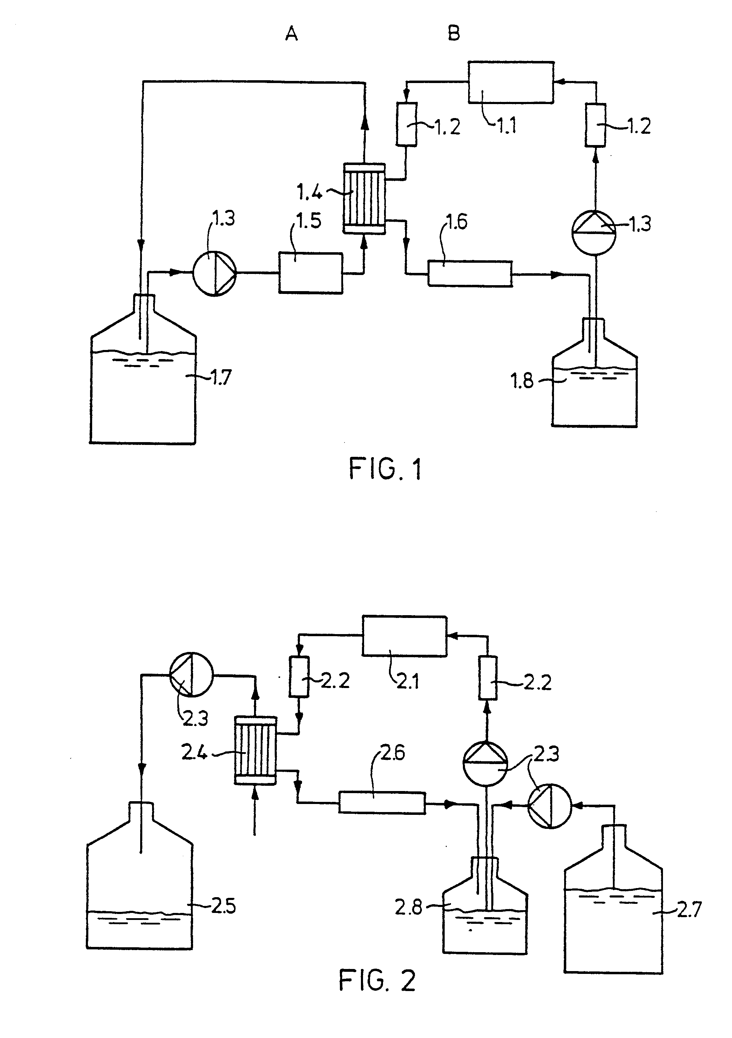

Das Fließschema ist in Abb. 1 zusammengefaßt. Es resultieren 2 getrennte Kreisläufe, der eigentliche Opticell®-Kreislauf, der z.B. durch den extrakapillaren Raum des Hohlfasermoduls geführt wird, und dem Dialyse- oder Austauschkreislauf, der im Gegenstrom durch die Hohlfasermembranen geführt wird. Das Verhältnis der Volumina beträgt für Opticell®-Medium zu Dialysemedium z.B. 1:10. Im Falle einer serumfreien Kultivierung enthält das Dialysemedium nur die dialysierbaren niedermolekularen Komponenten des Mediums.

Während der Dialyse reichern sich dialysierbare Stoffwechselendprodukte aus dem Opticell®-Medium ab, wobei gleichzeitig Substrate, die von den Zellen verbraucht werden, aus dem Austauschmedium nachtransportiert werden. Sind die Substrate des Austauschmediums erschöpft oder die Stoffwechselendprodukte zu schädigender Konzentration angereichert, muß dieses erneuert werden.

(b) <u>Ultrafiltration</u>

Das Fließschema ist in Abb. 2 zusammengefaßt. Das Hohlfasermodul wird zur Ultrafiltration verwendet. Ein Teil des zirkulierenden Opticell®-Mediums wird durch die Hohl-

<u>Le A 24 835</u>

fasermembranen absatzweise oder kontinuierlich abgezogen. Das Filtrat, das die niedermolekularen Komponenten und Stoffwechselprodukte enthält, wird verworfen. Entsprechend wird frisches Medium, das im Falle einer serumfreien Kultivierung nur ultrafiltrierbare Komponenten enthält, direkt ins Mediumreservoir des Opticell® zugeführt.

Wenn sich das Produkt im Opticell®-Medium genügend angereichert hat, wird es absatzweise oder kontinuierlich geerntet und das abgezogene Volumen durch frisches Medium ersetzt.

Le A 24 835

Beispiele

Beispiel 1

Kultivierung der humanen EBV-transformierte Zellinie
9D10

Produkt: monoklonale IgM Antikörper,

Medium: serumfreies Medium bestehend aus einer 1:1
Mischung der Basalmedien DMEM und HAM's F12 unter Zusatz
der Proteine HSA (humanes Serumalbumin), 2 g/1),
Transferrin (10 mg/1) und Insulin (10 mg/1) sowie
anderer niedermolekularer Supplemente. Das Austauschmedium für Dialyse oder Ultrafiltration enthielt nur die
niedermolekularen Supplemente.

Kulturbedingungen: $pO_2$ (am Eingang zur Wachstumskammer)
100 % Luftsättigung; pH (Eingang) 7.2-7.3; Temperatur
$37^0$ C; Pumpgeschwindigkeit im Opticell® 200-500 rpm,
letztere wurde so geregelt, daß der $pO_2$ am Ausgang der
Wachstumskammer nicht unter 40 % Luftsättigung absinkt.

Durchführung: die Kultur wurde mit $1.5 \times 10^9$ Zellen in 6
1 serumfreiem Medium gestartet. Als Wachstumskammer
diente Opticore S® zur Immobilisierung nicht-adhärenter
Zellen. Nach 139 h wurde die Dialyse mit 51 1 an Austauschmedium dazugeschaltet. Die Membran des verwendeten
Hohlfasermoduls hatte eine Molekulargewichtsausschlußgrenze von 3.000 bis 5.000 Dalton. Zur Produktgewinnung

Le A 24 835

wurden nach 259 h 1 l, nach 282 h 2,5 l und nach 306 h 1 l aus dem Opticell®geerntet und durch frisches Medium ersetzt. Nach 306 h wurde das Dialysemedium erneuert. In der nächsten Induktion wurden 1 l nach 330 h sowie 2 l nach 350 h geerntet. Nach 350 h wurde die Dialyse beendet und die Kultur auf "in line" Ultrafiltration umgestellt. Dies wurde nach 380 h mit einem Opticell® Volumen von 8 l gestartet. Zu Beginn betrug die Ultrafiltrationsrate 750 ml/h. Sie wurde nach 404 h auf 500 ml/h reduziert. Nach 474 h wurde die Kultur abgebrochen. Während der Ultrafiltrationsperiode wurden folgende Produkternten durchgeführt: 2 l nach 403 h, 1 l nach 427 h, 1 l nach 450 h und 8 l beim Abbruch. Die Konzentration des Proteinsupplement HSA wurde während der Kultivierung von 2 auf 0,6 g/l reduziert.

Analytik: Sauerstoffverbrauchsrate (OCR) der immobilisierten Zellen in der Opticore S®Wachstumskammer; Glukose- und IgM Konzentration im Kulturmedium.

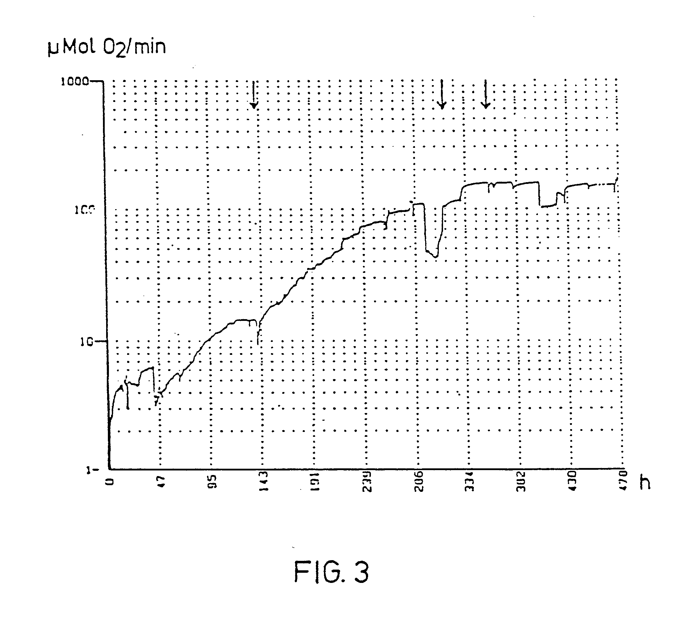

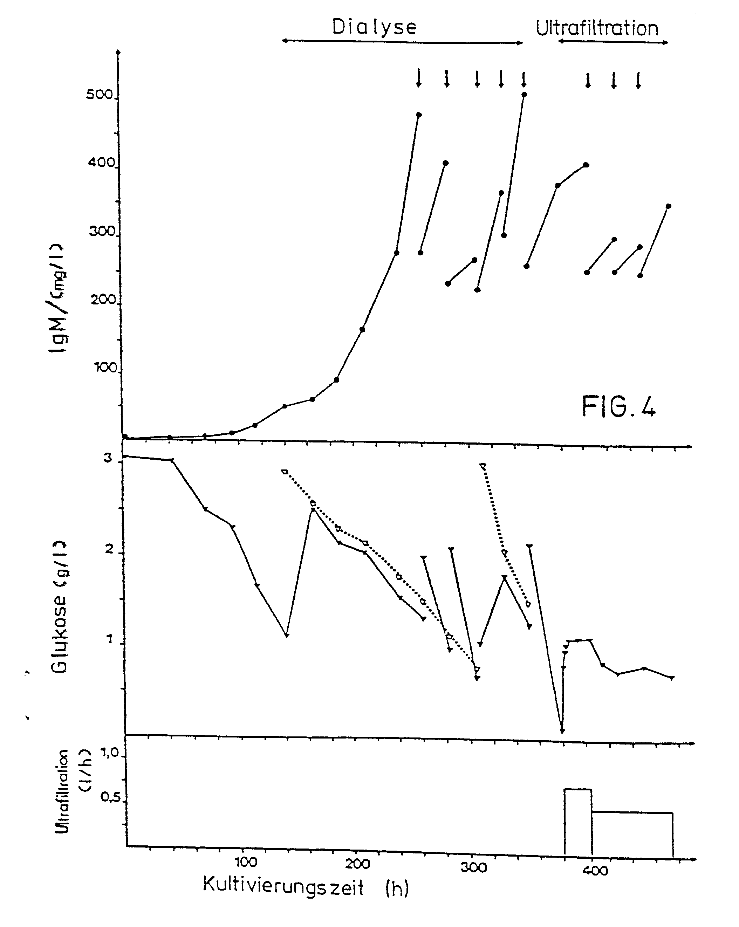

Ergebnis: Die Kinetik der OCR als Maßstab für das Wachstum und den Zustand der Zellen ist in Abb. 3 gezeigt; die Kinetik der IgM und Glukosekonzentration in Abb. 4.

Der Verlauf der OCR zeigt, daß sich die Zellen selbst in serumfreiem Medium vermehrten, bis die maximale Belegung der Wachstumskammer erreicht war. Danach stellte sich eine Gleichgewicht bei konstanter OCR ein.

Le A 24 835

- 11 -

Die Antikörperkonzentration im Opticell®-Medium stieg bis 500 mg/l an. Insgesamt wurden in 19,5 l Medium 7,5 g IgM geerntet. Die Produktionsraten waren von der aktuellen IgM Konzentration unabhängig, da die Zellen auch bei hoher Konzentration ihr Produkt unvermindert oder sogar noch schneller bildeten.

Anhand der Glukosekonzentration im Opticell®- und im Austauschmedium kann man verfolgen, daß die Dialyse während des gesamten Dialysezeitraums funktioniert hat. Während die Kultur ultrafiltriert wurde, stellten sich im Opticell® je nach Ultrafiltrationsrate Gleichgewichtsbedingungen ein, was wiederum durch eine konstante Glukosekonzentration angezeigt wird.

Vergleicht man die Opticell®-Kultur mit diskontinuierlichen Kulturen in Kulturflaschen (Tab. 1), so wurde die Produktkonzentration 10fach und die Produktbildungsraten pro Volumen an Kultursystem sogar bis zu 15fach erhöht.

Le A 24 835

Tab. 1: Vergleich der maximalen IgM Konzentration und der IgM Bildungsraten in Kulturflaschen und im Opticell® mit Dialyse oder Ultrafiltration

| Kultursystem/prozeß | max. IgM Konz. mg/l | IgM Produktions- rate mg/l/Tag |
|---|---|---|
| Opticell®, Dialyse | 520 | 150 |
| Opticell®, Ultrafiltrat. | 410 | 61 |
| Kulturflasche, diskont. | 50 | 10 |

**Beispiel 2**

**Kultivierung von Maushybridom H8**

**Produkt:** monoklonale IgG Antikörper,

**Medium:** serumfreies Medium bestehend aus einer 1:1 Mischung der Basalmedium DMEM und HAM's F12 unter Zusatz der Proteine HSA (2 g/l), Transferrin (10 mg/l) und Insulin (10 mg/l) sowie anderer niedermolekularer Supplemente. Das Austauschmedium zur Dialyse enthielt nur die niedermolekularen Supplemente.

**Kulturbedingungen:** $pO_2$ (am Eingang zur Wachstumskammer) 100-180 % Luftsättigung; pH (Eingang) 7.2-7.3; Temperatur 37° C; Pumpgeschwindigkeit im Opticell® 200-500 rpm; letztere wurde so geregelt, daß der $pO_2$ am Ausgang der Wachstumkammer nicht unter 30 % Luftsättigung absank.

**Le A 24 835**

Durchführung: die Kultur wurde mit $2.3 \times 10^9$ Zellen in 6 1 serumfreiem Medium gestartet. Als Wachstumskammer diente Opticore S$^®$ zu Immobilisierung nicht-adhärenter Zellen. Nach 41 h wurde die Dialyse mit 51 1 an Austauschmedium dazugeschaltet. Die Ausschlußgrenze der Dialysemembran betrug 3.000 bis 5.000 Dalton. Nach 139 h wurde das Dialysemedium erneuert sowie 3 1 Opticell$^®$-Medium geerntet und durch frisches Medium ersetzt. Diese Prozedur wurde in den nächsten 3 Induktionen wiederholt, wobei nach 208 und 304 h je 4,5 1 und nach 377 h 3 1 Opticell$^®$-Medium geerntet wurden. Die letzten beiden Induktionen wurden nach einem etwas anderen Schema gefahren. Hier wurden unabhängig vom Wechsel des Austauschmediums täglich 1-1,5 1 an produkthaltigem Opticell$^®$-Medium geerntet. Am Ende der Kultur nach 544 h wurden nochmals 6 1 Überstand erhalten. Das Volumen des Opticell$^®$-Mediums betrug 6-8 1. Die Konzentration des Proteinsupplement HSA wurde während der Kultivierung von 2 auf 0,2 g/1 reduziert.

Analytik: Sauerstoffverbrauchsrate (OCR) der immobilisierten Zellen in der Opticore S$^®$ Wachstumskammer; Glukose- und IgG Konzentration im Kulturmedium.

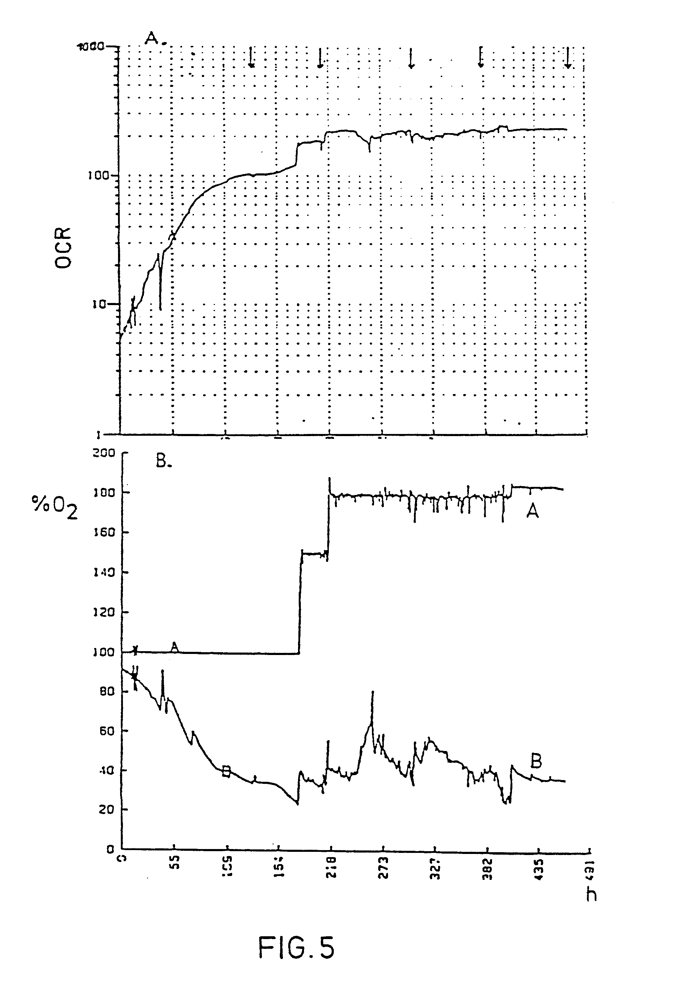

Ergebnis: Die Kinetiken der OCR als Maßstab für das Wachstum und den Zustand der Zellen sowie von $pO_2$ am Eingang und am Ausgang der Wachstumkammer sind in Abb. 5 gezeigt; die Kinetik der IgG und Glukosekonzentration in Abb. 5.

Le A 24 835

- 14 -

Der Verlauf der OCR zeigt, daß sich die Zellen in serumfreiem Medium vermehrten, bis die maximale Belegung der Wachstumskammer erreicht war. Danach stellten sich je nach dem $pO_2$ Wert im Medium verschiedene Gleichgewichts-OCR's ein.

Die Antikörperkonzentration im Opticell®-Medium stieg bis 2400 mg/l an. Da gleichzeitig die HSA-Konzentration reduziert wurde, betrug der Anteil des Produktes am Gesamtproteingehalt des Mediums am Ende 70-80 %. Insgesamt wurden in 26 l Medium 35 g IgG geerntet. Die Produktionsraten waren von der aktuellen IgG Konzentration unabhängig. Die Zellen bildeten ihr Produkt bei hoher Konzentration sogar eher besser.

Der Verlauf der Glukosekonzentration im Opticell®- und im Austauschmedium zeigt, daß die Dialyse während des gesamten Versuchs funktioniert hat.

Vergleicht man die Opticell®-Kultur mit diskontinuierlichen Kulturen in Kulturflaschen (Tab. 2), so wurde die Produktkonzentration 70fach und die Produktbildungsraten pro Volumen an Kultursystem sogar bis zu 160fach erhöht. Dies ist zum einen auf die kontrollierten Bedingungen (pH, $pO_2$) und zum anderen auf eine stark erhöhte Zellzahl zurückzuführen. So wurde am Ende des Versuchs mehr als $3 \times 10^{11}$ Zellen aus der Opticore S® Wachstumskammer geerntet. Bei einem Volumen von 6 l entspricht das einer

Le A 24 835

Konzentration von mehr als $5 \times 10^7$ Zellen pro ml Opticell®-Medium, was 30-40fach höher als in nicht-dialysierten Kulturen ist.

Tab. 2 Vergleich der maximalen IgG Konzentration und der IgG Bildungsraten in Kulturflaschen und im Opticell® mit Dialyse

| Kultursystem/prozeß | max. IgG Konz. mg/l | IgG Produktionsrate mg/l/Tag |
|---|---|---|
| Opticell®, Dialyse | 2400 | 650 |
| Kulturflasche, diskont. | 35 | 4 |

Le A 24 835

Legende zu den Zeichnungen

Fig. 1: Gleichgewichtsdialyse (Fließschema)

    A)    externer Kreislauf

    B)    interner Kreislauf

    1.1) Wachstumskammer für die Zellen

    1.2) Kontrolleinheiten für $pO_2$, pH, $pCO_2$ etc.

    1.3) Pumpen

    1.4) Dialysemodul

    1.5) Wärmeaustauscher

    1.6) Gasaustauscher zur Versorgung mit $O_2$ und Entsorgung von $CO_2$

    1.7) Reservoir für Austauschmedium

    1.8) internes Mediumreservoir

Fig. 2: Ultrafiltration (Fließschema)

    2.1) Wachstumskammer für die Zellen

    2.2) Kontrolleinheiten für $pO_2$, pH

    2.3) Pumpen

    2.4) Ultrafiltrationsmodul

    2.5) Abfallbehälter für verbrauchtes Medium

    2.6) Gasaustauscher zur Versorgung mit $O_2$ und Entsorgung von $CO_2$

    2.7) Behälter für frisches Medium

    2.8) internes Mediumreservoir

Fig. 3: Sauerstoffverbrauch (OCR) der Zellen in Abhängigkeit von der Kultivierungszeit (Beispiel 1)

Le A 24 835

Fig. 4: Konzentrationen IgM und Glucose in Abhängigvon der Kultivierungszeit (Beispiel 1)

——— Konzentration IgM

••• Konzentration Glucose im externen Kreislauf

——— Konzentration Glucose in internen Kreislauf

(↓) Ernte und Mediumaustausch

Fig. 5: Sauerstoffverbrauch und -sättigung in Abhängigkeit von der Kultivierungszeit (Beispiel 2) Kurve A zeigt die Sauerstoffsättigung am Eingang der Wachstumskammer und die
Kurve B zeigt die Sauerstoffsättigung am
Ausgang der Wachstumskammer.

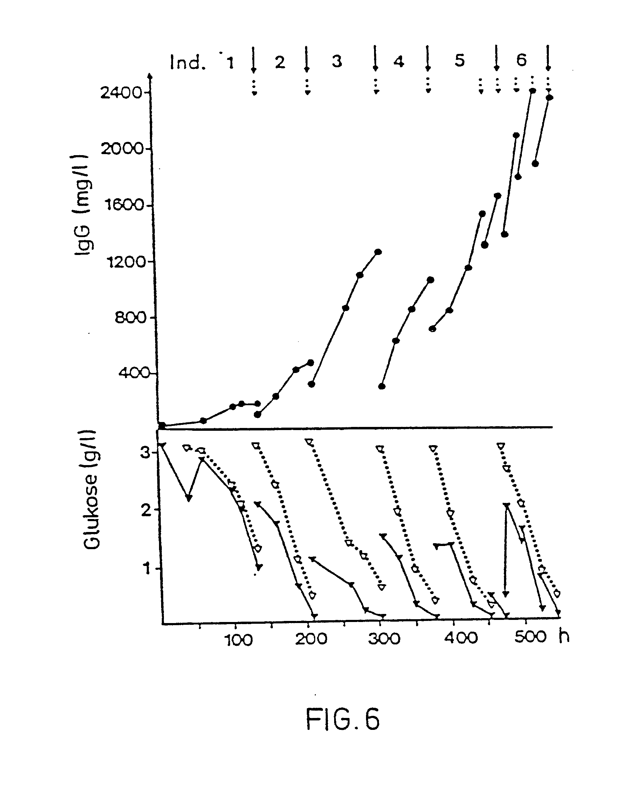

Fig. 6: Konzentration IgM und Glucose in Abhängigkeit
von der Kultivierungszeit (Beispiel 2)

——— Konzentration IgM

••• Konzentration Glucose im externen Kreislauf

——— Konzentration Glucose in internen Kreislauf

(↓) Ernte und Mediumaustausch

Le A 24 835

- 18 -

## Patentansprüche

1. Verfahren zur Kultivierung von immobilisierten oder adhärierter Zellen in einem Kultursystem, wobei die Zellen in einer Wachstumskammer immobilisiert oder adhäriert vorliegen und von im Kreislauf geführten Medium umspült sind, dadurch gekennzeichnet, daß die Zuführung von Nährstoffen und/oder die Abführung von Stoffwechselendprodukten kontinuierlich über eine separat im Kreislauf angeordnete semipermeable Membran erfolgt.

2. Verfahren nach Anspruch 1, worin die semipermeable Membran eine Dialysemembran ist und die Kultivierung der Zellen in einem an sich geschlossen Kultursystem stattfindet, welches über die Dialysemembran mit einen externen Mediumkreislauf verbunden ist, wobei die Zuführung von Nährstoffen und die Abführung von Stoffwechselendprodukten durch Dialyse erfolgt.

3. Verfahren nach Anspruch 1, worin die semipermeable Membran eine Ultrafiltrationsmembran ist und die Kultivierung der Zellen in einem an sich geschlossenen Kultursystem stattfindet, die Zuführung der Nährstoffe aus einem separaten Vorratsgefäß erfolgt und die Abführung der Stoffwechselendprodukte durch Ultrafiltration erfolgt.

4. Verfahren gemäß den Ansprüchen 1 bis 3, dadurch gekennzeichnet, daß die semipermeable Membran undurchlässig ist für Moleküle mit einem Molekulargewicht von mehr als 100 000 Dalton.

<u>Le A 24 835</u>

5. Verfahren gemäß den Ansprüchen 1 bis 3, dadurch gekennzeichnet, daß die semipermeable Membran undurchlässig ist für Moleküle mit einem Molekulargewicht von mehr als 50 000 Dalton.

6. Verfahren gemäß den Ansprüchen 1 bis 3, dadurch gekennzeichnet, daß die semipermeable Membran undurchlässig ist für Moleküle mit einem Molekulargewicht von mehr als 10 000 Dalton.

7. Vorrichtung zur Durchführung des Verfahrens gemäß den Ansprüchen 1 bis 6 bestehend aus einem Kultursystem für immobilisierte oder adhärierte Säugerzellen zusätzlich enthaltend ein Modul mit einer semipermeablen Membran.

8. Vorrichtung nach Anspruch 7 bei der das Modul mit der semipermeablen Membran an einen externen Mediumkreislauf angeschlossen ist und die Zuführung der Nährstoffe und die Abführung der Stoffwechselendprodukte durch Dialyse erfolgt.

9. Vorrichtung nach Anspruch 7 bei der das Kultursystem mit einem externen Mediumreservoir verbunden ist, aus dem die Nährstoffe zugeführt werden und über das Modul mit der semipermeablen Membran die Stoffwechselendprodukte durch Ultrafiltration abgeführt werden.

**Le A 24 835**

10. Verwendung der Vorrichtung gemäß den Ansprüchen 7 bis 9 bei der Herstellung von rekombinanten und natürlichen Proteinen, Enzymen und/oder Antikörpern.

Le A 24 835

FIG. 1

FIG. 2

0 270 905

FIG. 3

FIG. 4

0 270 905

FIG.5

FIG.6